# EUROPÄISCHE PATENTANMELDUNG

(11) **EP 0 895 994 A2**
(43) Veröffentlichungstag der Anmeldung: **10.02.1999**
(21) Anmeldenummer: 98114307.6
(22) Anmeldetag: 30.07.1998
(51) Int. Cl.: C07D 491/052, A61K 31/35, A61K 31/395

(54) **Sulfonamid-substituierte Pyranopyridine, Verfahren zu ihrer Herstellung, ihre Verwendung als Medikament oder Diagnostikum sowie sie enthaltendes Medikament**

(30) Priorität: 05.08.1997 DE 19733779; 30.10.1997 DE 19747889
(71) Anmelder: Hoechst Marion Roussel Deutschland GmbH, 65929 Frankfurt am Main (DE)
(72) Erfinder: Gerlach, Uwe, Dr., 65795 Hattersheim (DE); Lang, Hans Jochen, Dr., 65719 Hofheim (DE); Weidmann, Klaus, Dr., 61476 Kronberg (DE); Brendel, Joachim, Dr., 61118 Bad Vilbel (DE)

(57) **Zusammenfassung**

Verbindungen der Formel I 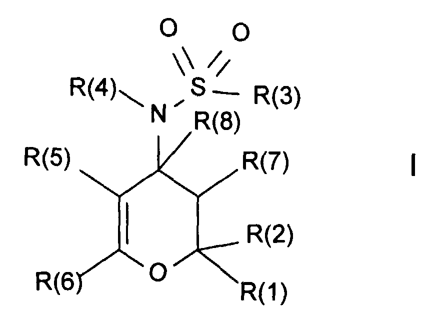 mit den in den Ansprüchen angebenen Bedeutungen der Substituenten sind hervorragend wirksame Substanzen zum Herstellen von Medikamenten zur Prophylaxe und zur Therapie von Herz-Kreislauferkrankungen, insbesondere Arrhythmien, zur Behandlung von Ulcera des Magen-Darm-Bereichs oder zur Behandlung von Durchfallerkrankungen.

## Beschreibung

Die Erfindung betrifft Verbindungen der Formel I 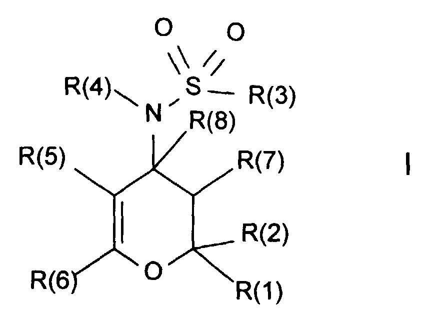 worin R(1), R(2), R(3), R(4), R(5), R(6), R(7) und R(8) die im folgenden angegebenen Bedeutungen haben, ihre Herstellung und ihre Verwendung, insbesondere in Arzneimitteln.

Die Verbindungen beeinflussen den durch cyclisches Adenosinmonophosphat (cAMP) geöffneten Kaliumkanal bzw. den I_{Ks}-Kanal und eignen sich in hervorragender Weise als Arzneimittelwirkstoffe, beispielsweise zur Prophylaxe und Therapie von Herz-Kreislauferkrankungen, insbesondere Arrhythmien, zur Behandlung von Ulcera des Magen-Darm-Bereichs oder zur Behandlung von Durchfallerkrankungen.

In der Arzneimittelchemie wurde in den letzten Jahren die Klasse der 4-Acylaminochroman-Derivate intensiv bearbeitet. Prominentester Vertreter dieser Klasse ist das Cromakalim der Formel A (J. Chem. Soc. Perkin Trans. 1, 1991, 63-70).

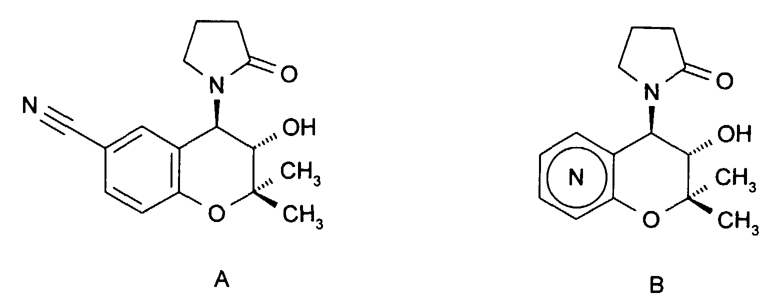

Bei Cromakalim und anderen verwandten 4-Acylaminochroman-Derivaten handelt es sich um Verbindungen mit relaxierender Wirkung auf glattmuskuläre Organe, so daß sie zur Senkung des erhöhten Blutdruckes infolge Gefäßmuskelrelaxation und in der Behandlung des Asthmas infolge der Relaxation der glatten Muskulatur der Atemwege verwendet werden. All diesen Präparaten ist gemeinsam, daß sie auf zellulärer Ebene beispielsweise von glatten Muskelzellen wirken und dort zu einer Öffnung bestimmter ATP-sensitiver K⁺-Kanäle führen. Der durch den Austritt von K⁺-Ionen induzierte Anstieg von negativer Ladung in der Zelle (Hyperpolarisation) wirkt über Sekundärmechanismen der Erhöhung der intrazellulären Ca²⁺-Konzentration und damit einer Zellaktivierung entgegen, die z. B. zu einer Muskelkontraktion führt.

Ähnliche Strukturen wie die der Formel I, die sogenannten Pyranopyridine (Formel B), sind in der Literatur beschrieben. Jedoch handelt es sich auch hier ausschließlich um 4-Acylamino-Derivate, die ebenfalls K-ATP-Kanal-blockierende Eigenschaften aufweisen.

Von diesen Acylamino-Derivaten unterscheiden sich die erfindungsgemäßen Verbindungen der Formel I strukturell unter anderem durch den Ersatz der Acylaminogruppe durch eine Sulfonylaminofunktion. Während das Cromakalim (Formel A) und analoge Acylaminoverbindungen als Öffner ATP-sensitiver K⁺-Kanäle wirken, zeigen die erfindungsgemäßen Verbindungen der Formel I mit der Sulfonylaminostruktur jedoch keine öffnende Wirkung auf diesen K⁺(ATP)-Kanal, sondern überraschenderweise eine starke und spezifische blockierende (schließende) Wirkung auf einen K⁺-Kanal, der durch cyclisches Adenosinmonophosphat (cAMP) geöffnet wird und sich grundlegend vom erwähnten K⁺(ATP)-Kanal unterscheidet. Neuere Untersuchungen zeigen, daß dieser an Dickdarmgewebe identifizierte K⁺(cAMP)-Kanal sehr ähnlich, vielleicht sogar identisch, mit dem am Herzmuskel identifizierten I_{Ks}-Kanal ist. In der Tat konnte für die erfindungsgemäßen Verbindungen der Formel I eine starke blockierende Wirkung auf den I_{Ks}-Kanal in Meerschweinchen-Cardiomyozyten wie auch auf den in Xenopus-Oozyten exprimierten I_{sK}-Kanal gezeigt werden. Infolge dieser Blockierung des K⁺(cAMP)-Kanals bzw. des I_{Ks}-Kanals entwickeln die erfindungsgemäßen Verbindungen im lebenden Organismus pharmakologische Wirkungen von hoher therapeutischer Verwertbarkeit.

Die Erfindung betrifft Verbindungen der Formel I 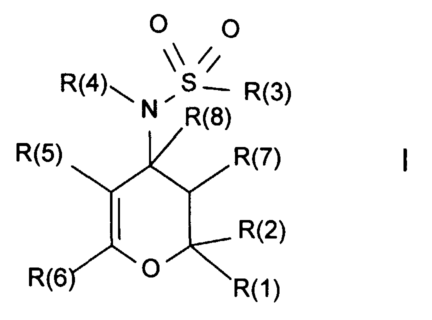 worin bedeuten:
R(1) und R(2)
   unabhängig voneinander Wasserstoff, CF₃, C₂F₅, C₃F₇, Alkyl mit 1, 2, 3, 4, 5 oder 6 C-Atomen oder Phenyl,
      das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
   oder
R(1) und R(2)
   gemeinsam eine Alkylenkette mit 2, 3, 4, 5, 6, 7, 8, 9 oder 10 C-Atomen;
      wobei eine CH₂-Gruppe der Alkylenkette durch -O-, -CO-, -S-, -SO-, -SO₂- oder -NR(10)- ersetzt sein kann;
         R(10) Wasserstoff oder Alkyl mit 1, 2 oder 3 C-Atomen;
R(3) R(12)-CₐH₂ₐ[NR(13)]ₘ-;
   R(12) Wasserstoff oder Cycloalkyl mit 3, 4, 5, 6, 7 oder 8 C-Atomen, CF₃, C₂F₅ oder C₃F₇;
      - a: Null, 1, 2, 3, 4, 5, 6, 7, 8, 9 oder 10;
      - m: Null oder 1;
   R(13) Wasserstoff oder Alkyl mit 1, 2, 3, 4, 5 oder 6 C-Atomen; oder
   R(12) und R(13)
      gemeinsam eine Alkylengruppe mit 4, 5, 6, 7 oder 8 C-Atomen,
         wobei eine CH₂-Gruppe der Alkylengruppe durch -O-, -[SO_{Null, 1 oder 2}]-, -CO- oder -NR(10)- ersetzt sein kann;
         R(10) Wasserstoff oder Alkyl mit 1, 2 oder 3 C-Atomen;

   R(4) R(14)-CᵣH₂ᵣ;
      - r: Null, 1, 2, 3, 4, 5, 6, 7, 8, 9, 10, 11, 12, 13, 14, 15, 16, 17, 18, 19 oder 20;

      R(14) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
         das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
         wobei eine CH₂-Gruppe der Gruppe CᵣH₂ᵣ ersetzt sein kann durch -O-, -CH=CH-, -C≡C-, -CO-, -CO-O-, -CO-NR(11)-, -[SO_{Null, 1 oder 2}]- oder -NR(11)-;
         R(11) Wasserstoff oder -(CₐH₂ₐ)-R(10);
         wobei eine CH₂-Gruppe der Gruppe CₐH₂ₐ ersetzt sein kann durch -O-, -CH=CH-, -C≡C-, -CO-, -CO-O-, -O-CO-, -S-,-SO-, -SO₂-, NR(10)- oder -CONR(10)-;
            R(10) Wasserstoff oder Alkyl mit 1, 2 oder 3 C-Atomen;
   oder
R(3) und R(4)
   gemeinsam eine Alkylenkette mit 3, 4, 5, 6, 7 oder 8 C-Atomen,
      wobei eine CH₂-Gruppe der Alkylenkette durch -O-, -[SO_{Null, 1 oder 2}]-, -CO- oder -NR(11)- ersetzt sein kann;
         R(11) Wasserstoff oder -(CₐH₂ₐ)-R(10),
         wobei eine CH₂-Gruppe der Gruppe CₐH₂ₐ ersetzt sein kann durch -O-, -CH=CH-, -C≡C-, -CO-, -CO-O-, -O-CO-, -S-, -SO-, -SO₂-, NR(10)- oder -CONR(10)-;
            R(10) Wasserstoff oder Alkyl mit 1, 2 oder 3 C-Atomen;
R(5) und R(6)
   -CR(15)=CR(16)-CR(17)=N-,
   -CR(15)=CR(16)-N=CR(17)-,
   -CR(15)=N-CR(17)=N-,
   -CR(15)=N-N=CR(17)-,
   -N=CR(16)-CR(17)=N- oder
   -S-CR(15)=CR(16)-;
   R(15), R(16) und R(17)
      unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, CN, CF₃, C₂F₅, C₃F₇, N₃, NO₂, -CONR(19)R(21), -COOR(21),
         R(22)-CₛH₂ₛ-Z- oder Phenyl,
         das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
      R(19) Wasserstoff oder Alkyl mit 1, 2 oder 3 C-Atomen;
      R(21) Wasserstoff, Methyl, Ethyl, Phenyl oder
         -CᵤH₂ᵤ-NR(19)R(20);
            wobei das Phenyl unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
               R(20)
               Wasserstoff oder Alkyl mit 1, 2 oder 3 C-Atomen;

         - u: 2 oder 3;
      R(22) Wasserstoff, Cycloalkyl mit 3, 4, 5, 6, 7 oder 8 C-Atomen,
         -COOR(21), CONR(19)R(21), Thienyl, Imidazolyl, Pyridyl, Chinolyl, Isochinolyl, Piperidyl, 1-Pyrrolidinyl, N-Morpholino, N-Methylpiperazino, CF₃, C₂F₅, C₃F₇ oder Phenyl,
            das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;

         - s: Null, 1, 2, 3, 4, 5 oder 6;
         - Z: -[S(O)_{Null, 1 oder 2}]-, -CO-, -SO_{(0, 1 oder 2)}-NR(11)-, -SO₂-O-, -O-, -NR(11)- oder -[CO-NR(11)]-;
R(7) Wasserstoff, Hydroxy, Alkoxy mit 1, 2, 3 oder 4 C-Atomen, Acyloxy mit 1, 2, 3 oder 4 C-Atomen, Cl, Br, F, Alkyl mit 1, 2, 3 oder 4 C-Atomen;
R(8) Wasserstoff oder Alkyl mit 1, 2, 3, 4, 5 oder 6 C-Atomen;
   und deren physiologisch verträgliche Salze.

Bevorzugt sind Verbindungen der Formel I, in welcher bedeuten:
R(1) und R(2)
   unabhängig voneinander Wasserstoff, CF₃, C₂F₅, C₃F₇, Alkyl mit 1, 2, 3, 4, 5 oder 6 C-Atomen oder Phenyl,
      das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
   oder
R(1) und R(2)
   gemeinsam eine Alkylenkette mit 2, 3, 4, 5, 6, 7, 8, 9 oder 10 C-Atomen;
      wobei eine CH₂-Gruppe der Alkylenkette durch -O-,-CO-, -S-, -SO-, -SO₂- oder -NR(10)- ersetzt sein kann;
         R(10) Wasserstoff oder Alkyl mit 1, 2 oder 3 C-Atomen;
R(3) R(12)-CₐH₂ₐ[NR(13)]ₘ-;
   R(12) Wasserstoff oder Cycloalkyl mit 3, 4, 5, 6, 7 oder 8 C-Atomen,
      CF₃, C₂F₅ oder C₃F₇;

      - a: Null, 1, 2, 3, 4, 5, 6, 7, 8, 9 oder 10;
      - m: Null oder 1;
   R(13) Wasserstoff oder Alkyl mit 1, 2, 3, 4, 5 oder 6 C-Atomen;
R(4) R(14)-CᵣH₂ᵣ;
   - r: Null, 1, 2, 3, 4, 5, 6, 7, 8, 9, 10, 11, 12, 13, 14, 15, 16, 17, 18, 19 oder 20;
R(14) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
   das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
      wobei eine CH₂-Gruppe der Gruppe CᵣH₂ᵣ ersetzt sein kann durch -O-, -CH=CH-, -C≡C-, -CO-, -CO-O-, -CO-NR(11)-, -[SO_{Null, 1 oder 2}]- oder -NR(11)-;
      R(11) Wasserstoff oder -(CₐH₂ₐ)-R(10)
      wobei eine CH₂-Gruppe der Gruppe CₐH₂ₐ ersetzt sein kann durch -O-, -CH=CH-, -C≡C-, -CO-, -CO-O-, -O-CO-, -S-, -SO-, -SO₂-, NR(10)- oder -CONR(10)-;
         - a: Null, 1, 2, 3, 4, 5, 6, 7, 8, 9 oder 10;
R(5) und R(6)
   -CR(15)=CR(16)-CR(17)=N-,
   -CR(15)=CR(16)-N=CR(17)-,
   -CR(15)=N-CR(17)=N-,
   -CR(15)=N-N=CR(17)-,
   -N=CR(16)-CR(17)=N- oder
   -S-CR(15)=CR(16)-;
   R(15), R(16), R(17)
      unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, CN, CF₃, C₂F₅, C₃F₇, N₃, NO₂, -CONR(19)R(21), -COOR(21),
         R(22)-CₛH₂ₛ-Z- oder Phenyl,
         das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
      R(19) Wasserstoff oder Alkyl mit 1, 2 oder 3 C-Atomen;
      R(21) Wasserstoff, Methyl, Ethyl, Phenyl oder -CᵤH₂ᵤ-NR(19)R(20);
         - u: 2 oder 3;

         R(20) Wasserstoff oder Alkyl mit 1, 2 oder 3 C-Atomen; wobei das Phenyl unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
      R(22) Wasserstoff, Cycloalkyl mit 3, 4, 5, 6, 7 oder 8 C-Atomen,
         -COOR(21), CONR(19)R(21), Thienyl, Imidazolyl, Pyridyl, Chinolyl, Isochinolyl, Piperidyl, 1-Pyrrolidinyl, N-Morpholino, N-Methylpiperazino, CF₃, C₂F₅ oder C₃F₇ oder Phenyl,
            das unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;

      - s: Null, 1, 2, 3, 4, 5 oder 6;
      - Z: -[S(O)_{Null, 1 oder 2}]-, -CO-, -SO₂-NR(11)-, -SO₂-O-, -O-, -NR(11)- oder -[CO-NR(11)]-;
R(7) Wasserstoff, Hydroxy, Alkoxy mit 1, 2, 3 oder 4 C-Atomen, Acyloxy mit 1, 2, 3 oder 4 C-Atomen, Cl, Br, F, Alkyl mit 1, 2, 3 oder 4 C-Atomen;
R(8) Wasserstoff oder Alkyl mit 1, 2, 3, 4, 5 oder 6 C-Atomen;
   und deren physiologisch verträgliche Salze.

Besonders bevorzugt sind Verbindungen der Formel I, in der bedeuten:
R(1) und R(2)
   unabhängig voneinander Wasserstoff, CF₃, C₂F₅, C₃F₇, Alkyl mit 1, 2, 3, 4, 5 oder 6 C-Atomen oder Phenyl,
      das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
   oder
R(1) und R(2)
   gemeinsam eine Alkylenkette mit 2, 3, 4, 5, 6, 7, 8, 9 oder 10 C-Atomen;
      wobei eine CH₂-Gruppe der Alkylenkette durch -O-, -CO-, -S-, -SO-, -SO₂- oder -NR(10)- ersetzt sein kann;
         R(10) Wasserstoff oder Alkyl mit 1, 2 oder 3 C-Atomen;
R(3) R(12)-CₐH₂ₐ[NR(13)]ₘ-;
   R(12) Wasserstoff oder Cycloalkyl mit 3, 4, 5, 6, 7 oder 8 C-Atomen, CF₃, C₂F₅ oder C₃F₇;
      - a: Null, 1, 2, 3, 4, 5, 6, 7, 8, 9 oder 10;
      - m: Null oder 1;
   R(13) Wasserstoff oder Alkyl mit 1, 2, 3, 4, 5 oder 6 C-Atomen;
R(4) R(14)-CᵣH₂ᵣ;
   - r: 1, 2, 3, 4, 5, 6, 7, 8, 9, 10, 11, 12, 13, 14, 15, 16, 17, 18, 19 oder 20;

   R(14) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
      das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
   wobei eine CH₂-Gruppe der Gruppe CᵣH₂ᵣ ersetzt sein kann durch
   -O-, -CH=CH-, -C≡C-, -CO-, -CO-O-, -CO-NR(11)-, -[SO_{Null, 1 oder 2}]- oder -NR(11)-;
   R(11) Wasserstoff oder -(CₐH₂ₐ)-R(10),
      wobei eine CH₂-Gruppe der Gruppe CₐH₂ₐ ersetzt sein kann durch -O-, -CH=CH-, -C≡C-, -CO-, -CO-O-, -O-CO-, -S-, -SO-, -SO₂-, NR(10)- oder -CONR(10)-;
R(5) und R(6)
   -CR(15)=CR(16)-CR(17)=N- oder
   -CR(15)=CR(16)-N=CR(17)-;
   R(15), R(16), R(17)
      unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, CN, CF₃, C₂F₅, C₃F₇, N₃, NO₂, -CONR(19)R(21), -COOR(21),
      R(22)-CₛH₂ₛ-Z- oder Phenyl,
         das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
      R(19) Wasserstoff oder Alkyl mit 1, 2 oder 3 C-Atomen;
      R(21) Wasserstoff, Methyl, Ethyl, Phenyl oder
         -CᵤH₂ᵤ-NR(19)R(20);
            - u: 2 oder 3;

            R(20) Wasserstoff oder Alkyl mit 1, 2 oder 3 C-Atomen;
               wobei das Phenyl unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
         R(22) Wasserstoff, Cycloalkyl mit 3, 4, 5, 6, 7 oder 8 C-Atomen,
            -COOR(21), CONR(19)R(21), Thienyl, Imidazolyl, Pyridyl, Chinolyl, Isochinolyl, Piperidyl, 1-Pyrrolidinyl, N-Morpholino, N-Methylpiperazino, CF₃, C₂F₅ oder C₃F₇ oder Phenyl,
               das unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;

         - s: Null, 1, 2, 3, 4, 5 oder 6;
         - Z: -[S(O)_{Null, 1 oder 2}]-, -CO-, -SO₂-NR(11)-, -SO₂-O-, -O-, -NR(11)- oder -[CO-NR(11)]-;
R(7) Wasserstoff, Hydroxy, Alkoxy mit 1, 2, 3 oder 4 C-Atomen, Alkyl mit 1, 2, 3 oder 4 C-Atomen;
R(8) Wasserstoff oder Alkyl mit 1, 2, 3, 4, 5 oder 6 C-Atomen;
   und deren physiologisch verträgliche Salze.

Ganz besonders bevorzugt sind Verbindungen der Formel I, in der bedeuten:
R(1) und R(2)
   unabhängig voneinander Wasserstoff, CF₃, C₂F₅, C₃F₇, Alkyl mit 1, 2, 3, 4, 5 oder 6 C-Atomen oder Phenyl,
      das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
   oder
R(1) und R(2)
   gemeinsam eine Alkylenkette mit 2, 3, 4, 5, oder 6 C-Atomen;
      wobei eine CH₂-Gruppe der Alkylenkette durch -O-, -CO-, -S-, -SO-, -SO₂- oder -NR(10)- ersetzt sein kann;
      R(10) Wasserstoff oder Alkyl mit 1, 2 oder 3 C-Atomen;
R(3) R(12)-CₐH₂ₐ-;
   R(12) Wasserstoff oder Cycloalkyl mit 3, 4, 5, 6, 7 oder 8 C-Atomen,
      CF₃, C₂F₅ oder C₃F₇;
         - a: Null, 1, 2, 3, 4, 5, 6, 7, 8, 9 oder 10;
   R(4) R(14)-CᵣH₂ᵣ;
      - r: 1, 2, 3, 4, 5, 6, 7, 8, 9, 10, 11, 12, oder 13;
      R(14) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
         das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
         wobei eine CH₂-Gruppe der Gruppe CᵣH₂ᵣ ersetzt sein kann durch -O-, -CH=CH-, -C≡C-, -CO-, -CO-O-, -CO-NR(11)-, -[SO_{Null, 1 oder 2}]- oder -NR(11)-;
         R(11) Wasserstoff oder -(CₐH₂ₐ)-R(10)
         wobei eine CH₂-Gruppe der Gruppe CₐH₂ₐ ersetzt sein kann durch -O-, -CH=CH-, -C≡C-, -CO-, -CO-O-, -O-CO-, -S-, -SO-, -SO₂-, NR(10)- oder -CONR(10)-;
            R(10) Wasserstoff oder Alkyl mit 1, 2 oder 3 C-Atomen;
R(5) und R(6)
   -CR(15)=CR(16)-CR(17)=N- oder
   -CR(15)=CR(16)-N=CR(17)-;
   R(15), R(16), R(17)
      unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, CN, CF₃, C₂F₅, C₃F₇, N₃, NO₂, -CONR(19)R(21), -COOR(21),
      R(22)-CₛH₂ₛ-Z- oder Phenyl,
         das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
      R(19)
         Wasserstoff oder Alkyl mit 1, 2 oder 3 C-Atomen;
      R(21) Wasserstoff, Methyl, Ethyl oder Phenyl,
         wobei das Phenyl unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
      R(22) Wasserstoff;
         - s: Null, 1, 2, 3, 4, 5 oder 6;
         - Z: -[S(O)_{Null, 1 oder 2}]-, -CO-, -SO₂-NR(11)-, -SO₂-O-, -O-, -NR(11)- oder -[CO-NR(11)]-;
R(7) Wasserstoff, Hydroxy, Alkoxy mit 1, 2, 3 oder 4 C-Atomen, Alkyl mit 1, 2, 3 oder 4 C-Atomen;
R(8) Wasserstoff oder Alkyl mit 1, 2, 3, 4, 5 oder 6 C-Atomen;
   und deren physiologisch verträgliche Salze.

Enthalten die Verbindungen I eine saure oder basische Gruppe bzw. einen basischen Heterocyclus, so sind auch die entsprechenden, pharmakologisch und toxikologisch verträglichen Salze Gegenstand der Erfindung. So können die Verbindungen I, die eine oder mehrere -COOH-Gruppen tragen, beispielsweise als Alkalisalze, vorzugsweise als Natrium- oder Kaliumsalze verwendet werden. Verbindungen I, die eine basische, protonierbare Gruppe oder einen basischen heterocyclischen Rest tragen, können auch in Form ihrer organischen oder anorganischen, pharmakologisch und toxikologisch verträglichen Säureadditionssalze verwendet werden, beispielweise als Hydrochloride, Methansulfonate, Acetate, Lactate, Maleinate, Fumarate, Malate, Gluconate usw. Enthalten die Verbindungen I eine saure und basische Gruppe im gleichen Molekül, so gehören neben den geschilderten Salzformen auch innere Salze, sogenannte Betaine, zur der Erfindung.

Die Verbindungen der Formel I können bei entsprechender Substitution in stereoisomeren Formen vorliegen. Enthalten die Verbindungen der Formel I ein oder mehrere Asymmetriezentren, so können diese unabhängig voneinander die S-Konfiguration oder die R-Konfiguration aufweisen. Zur Erfindung gehören alle möglichen Stereoisomeren, z. B. Enantiomere oder Diastereomere, und Mischungen von zwei oder mehr stereoisomeren Formen, z. B. Enantiomeren und/oder Diastereomeren, in beliebigen Verhältnissen. Enantiomere z. B. sind also in enantiomerenreiner Form, sowohl als links- als auch als rechtsdrehende Antipoden, und auch in Form von Mischungen der beiden Enantiomeren in unterschiedlichen Verhältnissen oder in Form von Racematen Gegenstand der Erfindung. Bei Vorliegen einer cis/trans-Isomerie sind sowohl die cis-Form als auch die trans-Form und Gemische dieser Formen Gegenstand der Erfindung. Die Herstellung von einzelnen Stereoisomeren kann gewünschtenfalls durch Auftrennung eines Gemisches nach üblichen Methoden oder z. B. durch stereoselektive Synthese erfolgen. Bei Vorliegen von beweglichen Wasserstoffatomen umfaßt die vorliegende Erfindung auch alle tautomeren Formen der Verbindungen der Formel I.

Alkyl- und Alkylenreste können geradkettig oder verzweigt sein.

Die Verbindungen der Formel I sind durch unterschiedliche chemische Verfahren herstellbar, die ebenfalls Teil der Erfindung sind.

So erhält man eine Verbindung der Formel I, indem man
a) eine Verbindung der Formel II 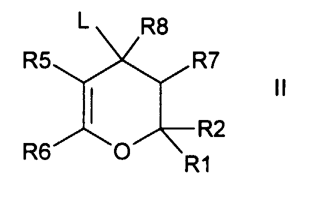 worin R(1), R(2), R(5), R(6), R(7) und R(8) die angegebene Bedeutung besitzen und L eine übliche nucleofuge Fluchtgruppe, insbesondere F, Cl, Br, I, MeSO₂-O-, einen p-Toluolsulfonyloxy-Rest, oder R(7) und L gemeinsam ein Epoxidring sind, bedeutet,
   mit einem Sulfonamid oder dessen Salz der Formel III 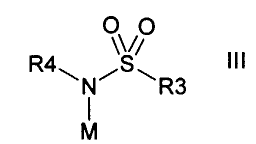 in an sich bekannter Weise umsetzt, worin R(3) und R(4) die angegebene Bedeutung besitzen und M für Wasserstoff oder vorzugsweise für ein Metalläquivalent, besonders bevorzugt für Lithium, Natrium oder Kalium steht;
   oder daß man
b) eine Verbindung der Formel IV 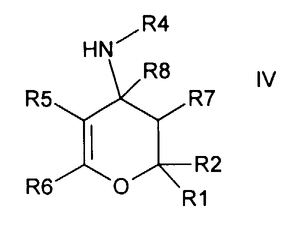 worin R(1), R(2), R(4), R(5), R(6), R(7) und R(8) die angegebene Bedeutung besitzen,
   mit einem Sulfonsäure-Derivat der Formel V 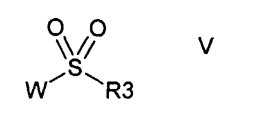 umsetzt, worin R(3) die angegebene Bedeutung besitzt und W eine nucleofuge Fluchtgruppe, wie Fluor, Brom, 1-Imidazolyl, insbesondere aber Chlor bedeutet;
   oder daß man
c) eine Verbindung der Formel VI 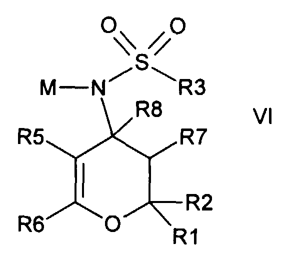 worin R(1), R(2), R(3), R(5), R(6), R(7), R(8), und M die angegebene Bedeutung besitzen, in an sich bekannter Weise mit einem Alkylierungsmittel der Formel VII

   R(4)-L VII

   im Sinne einer Alkylierungsreaktion umsetzt, worin R(4) mit Ausnahme von Wasserstoff sowie L die angegebene Bedeutung besitzen;
   oder daß man
d) in einer Verbindung der Formel I 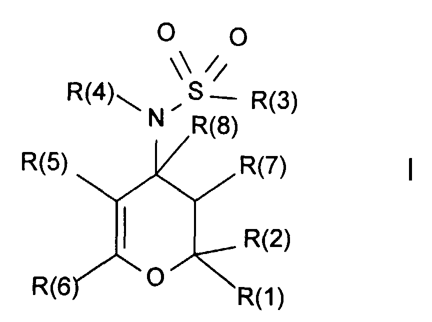 worin R(1) bis R(4), R(7) und R(8) die angegebene Bedeutung besitzen, in mindestens einer der Positionen R(15), R(16), R(17) des Ringsystems R(5)-R(6) eine elektrophile Substitutionsreaktion durchführt, sofern diese Position Wasserstoff bedeutet und die restlichen Substituenten R(1) bis R(8) die angegebene Bedeutung besitzen.

### Verfahrensweise a)

beschreibt die Reaktion eines Sulfonamids bzw. eines seiner Salze der Formel III mit einem reaktiven Heterocyclus der Formel II. Da die Reaktion eines Sulfonamides III aus der Salzform heraus erfolgt, muß bei Verwendung eines freien Sulfonamids (Formel III, M = H) durch Einwirkung einer Base ein Sulfonamidsalz (Formel III, M = Kation) erzeugt werden, das sich durch höhere Nucleophilie und damit durch höhere Reaktivität auszeichnet. Setzt man freies Sulfonamid (M = H) ein, so erfolgt die Deprotonierung des Sulfonamids zum Salz in situ unter bevorzugter Verwendung solcher Basen, die selbst nicht oder nur wenig alkyliert werden, wie Natriumcarbonat, Kaliumcarbonat, ein sterisch stark gehindertes Amin, z. B. Dicyclohexylamin, N,N,N-Dicyclohexyl-ethylamin oder andere starke Stickstoffbasen mit geringer Nucleophilie, beispielsweise DBU, N,N',N'''-Triisopropylguanidin etc. Es können allerdings auch andere üblich verwendete Basen für die Reaktion eingesetzt werden, wie Kalium-tert.butylat, Natriummethylat, Alkalihydrogencarbonate, Alkalihydroxide, wie beispielweise LiOH, NaOH oder KOH, oder Erdalkalihydroxide, beispielsweise Ca(OH)₂.

Dabei arbeitet man vorzugsweise in polaren organischen Lösungsmitteln wie Dimethylformamid, Dimethylacetamid, Tetramethylharnstoff, Hexamethylphosphorsäuretriamid, Tetrahydrofuran, Dimethoxyethan, Toluol, einem halogenierten Kohlenwasserstoff wie Chloroform oder Methylenchlorid usw. Prinzipiell kann aber auch in polaren protischen Lösungsmitteln gearbeitet werden, wie Wasser, Methanol, Ethanol, Isopropanol, Ethylenglykol oder dessen Oligomeren und deren entsprechenden Halbethern und Ethern. Die Reaktion wird in einem bevorzugten Temperaturbereich von -10 bis 140°C, besonders bevorzugt von 20 bis 100°C durchgeführt. In günstiger Weise kann Verfahrensweise a) auch unter den Bedingungen einer Zweiphasenkatalyse durchgeführt werden.

Die Verbindungen der Formel II gewinnt man nach literaturbekannten Methoden, beispielsweise aus den entsprechenden ungesättigten Verbindung X 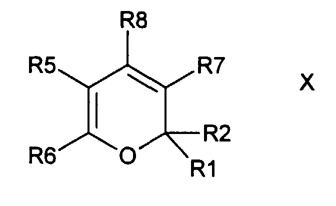 durch Einwirkung eines anorganischen oder organischen Peroxides, wie beispielsweise H₂O₂, MCPBA, Peressigsäure. Die Anlagerung von Halogen/OH ist auch durch die Reaktion von X mit NCS, NBS, Chlor oder Brom in wäßrigen Lösungsmitteln möglich. Im Falle des Epoxids (R(7) und L bilden einen Epoxidring) ist dieses aus dem Hydro-Halogenid durch Abspaltung von H-Halogen mit verschiedenen Basen darstellbar. Vorteilhaft arbeitet man in einem Lösungsmittel, das ausreichend inert gegenüber diesen halogenierenden oder oxidierenden Reagenzien sind, wie beispielsweise in DMSO oder halogenierten Kohlenwasserstoffen wie z.B. Chloroform, Methylenchlorid.

### Verfahrensweise b)

beschreibt die an sich bekannte und häufig angewandte Reaktion einer reaktiven Sulfonylverbindung der Formel V, insbesondere einer Chlorsulfonylverbindung (W = Cl), mit einem Amino-Derivat der allgemeinen Formel IV zum entsprechenden Sulfonamid-Derivat der allgemeinen Formel I. Die Reaktion kann prinzipiell ohne Lösungsmittel durchgeführt werden, jedoch werden derartige Reaktionen in den meisten Fällen unter Verwendung eines Lösungsmittels durchgeführt.

Die Reaktionsführung geschieht vorzugsweise unter Verwendung eines polaren Lösungsmittels vorzugsweise in Gegenwart einer Base, die selbst vorteilhaft als Lösungsmittel verwendet werden kann, z.B. bei Verwendung von Triethylamin, insbesondere von Pyridin und dessen Homologen. Ebenfalls verwendete Lösungsmittel sind beispielsweise Wasser, aliphatische Alkohole, z.B. Methanol, Ethanol, Isopropanol, sek. Butanol, Ethylenglykol und dessen monomere und oligomere Monoalkyl- und Dialkylether, Tetrahydrofuran, Dioxan, dialkylierte Amide wie DMF, DMA, sowie TMU und HMPT. Man arbeitet dabei bei einer Temperatur von 0 bis 160°C, vorzugsweise von 20 bis 100°C. Die Amino-Derivate der Formel IV erhält man in an sich literaturbekannter Weise bevorzugt durch Reaktion der reaktiven Verbindungen der Formel II mit R(1), R(2), R(5), R(6) und L in der angegebenen Bedeutung, entweder mit Ammoniak oder einem Amin der Formel XI

R(4)-NH₂ XI

mit R(4) in der angegebenen Bedeutung.

### Verfahrensweise c)

repräsentiert die an sich bekannte Alkylierungsreaktion eines Sulfonamids bzw. eines seiner Salze VI mit einem Alkylierungsmittel der Formel VII. Entsprechend der Reaktionsanalogie mit Verfahrensweise a) gelten für Verfahrensweise c) die bereits ausführlich unter Verfahrensweise a) beschriebenen Reaktionsbedingungen.

Die Herstellung der Sulfonamid-Derivate VI und deren Vorprodukte wurde bereits bei Verfahrensweise b) beschrieben. Die Herstellung der Alkylantien VII erfolgt nach Analogvorschriften der Literatur bzw. wie unter Verfahrensweise a) beschrieben, vorzugsweise aus den entsprechenden Hydroxyverbindungen (Formel VII mit L gleich -OH).

### Verfahrensweise d)

beschreibt die weitere chemische Umwandlung von erfindungsgemäßen Verbindungen der Formel I in andere Verbindungen der Formel I durch elektrophile Substitutionsreaktionen in einer oder in mehreren der mit R(5) bis R(8) bezeichneten Positionen, die jeweils Wasserstoff bedeuten. Bevorzugte Substitutionsreaktionen sind
1. die aromatische Nitrierung zur Einführung einer oder mehrerer Nitrogruppen, sowie deren nachfolgende Reduktion zu NH₂-,
2. die aromatische Halogenierung insbesondere zur Einführung von Chlor, Brom oder Jod,
3. die Chlorsulfonierung zur Einführung einer Chlorsulfonylgruppe durch Einwirkung von Chlorsulfonsäure,
4. die Friedel-Crafts Acylierungsreaktion zur Einführung eines Acylrestes nach literaturbekannten Methoden.

Bei allen Verfahrensweisen kann es angebracht sein, bei bestimmten Reaktionsschritten funktionelle Gruppen im Molekül zeitweilig zu schützen. Solche Schutzgruppentechniken sind dem Fachmann geläufig. Die Auswahl einer Schutzgruppe für in Betracht kommende Gruppen und die Verfahren zu ihrer Einführung und Abspaltung sind in der Literatur beschrieben und können gegebenenfalls ohne Schwierigkeiten dem Einzelfall angepaßt werden.

Es wurde bereits gesagt, daß die Verbindungen der Formel I überraschenderweise eine starke und spezifische blockierende (schließende) Wirkung auf einen K⁺-Kanal haben, der durch cyclisches Adenosinmonophosphat (cAMP) geöffnet wird und sich grundlegend vom wohlbekannten K⁺(ATP)-Kanal unterscheidet, und daß dieser an Dickdarmgewebe identifizierte K⁺(cAMP)-Kanal sehr ähnlich, vielleicht sogar identisch, mit dem am Herzmuskel identifizierten I_{Ks}-Kanal ist. Für die erfindungsgemäßen Verbindungen konnte eine starke blockierende Wirkung auf den I_{Ks}-Kanal in Meerschweinchen-Cardiomyozyten wie auch auf den in Xenopus-Oozyten exprimierten I_{sK}-Kanal gezeigt werden. Infolge dieser Blockierung des K⁺(cAMP)-Kanals bzw. des I_{Ks}-Kanals entwickeln die erfindungsgemäßen Verbindungen im lebenden Organismus pharmakologische Wirkungen von hoher therapeutischer Verwertbarkeit und eignen sich in hervorragender Weise als Arzneimittelwirkstoffe für die Therapie und Prophylaxe verschiedener Krankheitsbilder.

So zeichnen sich die erfindungsgemäßen Verbindungen der Formel I als neue Wirkstoffklasse potenter Inhibitoren der stimulierten Magensäuresekretion aus. Die Verbindungen der Formel I sind somit wertvolle Arzneimittelwirkstoffe zur Therapie und Prophylaxe von Ulcera des Magens und des intestinalen Bereiches, beispielsweise des Duodenums. Sie eignen sich ebenfalls infolge ihrer starken magensaftsekretionshemmenden Wirkung als ausgezeichnete Therapeutika zur Therapie und Prophylaxe der Refluxösophagitis.

Die erfindungsgemäßen Verbindungen der Formel I zeichnen sich weiterhin durch eine antidiarrhoische Wirkung aus und sind deshalb als Arzneimittelwirkstoffe zur Therapie und Prophylaxe von Durchfallerkrankungen geeignet.

Weiterhin eignen sich die erfindungsgemäßen Verbindungen der Formel I als Arzneimittelwirkstoffe zur Therapie und Prophylaxe von Herz-Kreislauferkrankungen. Insbesondere können sie zur Therapie und Prophylaxe aller Typen von Arrhythmien verwendet werden, einschließlich atrialer, ventrikulärer und supraventrikulärer Arrhythmien, vor allem von Herzrhythmusstörungen, die durch Aktionspotential-Verlängerung behoben werden können. Sie können speziell angewandt werden zur Therapie und Prophylaxe von atrialer Fibrillation (Vorhofflimmern) und atrialem Flattern (Vorhofflattern) sowie zur Therapie und Prophylaxe von Reentry-Arrhythmien und zur Verhinderung des plötzlichen Herztodes infolge von Kammerflimmern.

Obwohl bereits zahlreiche antiarrhythmisch wirkende Substanzen auf dem Markt sind, gibt es doch keine Verbindung, die hinsichtlich Wirksamkeit, Anwendungsbreite und Nebenwirkungsprofil wirklich zufriedenstellend ist, so daß weiterhin eine Notwendigkeit zur Entwicklung verbesserter Antiarrhythmika besteht.
Die Wirkung zahlreicher bekannter Antiarrhythmika der sogenannten Klasse III beruht auf einer Erhöhung der myocardialen Refraktärzeit durch Verlängerung der Aktionspotentialdauer. Diese wird im wesentlichen bestimmt durch das Ausmaß repolarisierender K⁺-Ströme, die über verschiedene K⁺-Kanäle aus der Zelle herausfließen. Eine besonders große Bedeutung wird hierbei dem sogenannten "delayed rectifier" I_{K} zugeschrieben, von dem zwei Subtypen existieren, ein schnell aktivierter I_{Kr} und ein langsam aktivierter I_{Ks}. Die meisten bekannten Klasse III- Antiarrhythmika blockieren überwiegend oder ausschließlich I_{Kr} (z.B. Dofetilid, d-Sotalol). Es hat sich jedoch gezeigt, daß diese Verbindungen bei geringen oder normalen Herzfrequenzen ein erhöhtes proarrhythmisches Risiko aufweisen, wobei insbesondere Arrhythmien, die als "Torsades de pointes" bezeichnet werden, beobachtet wurden (D.M. Roden; "Current Status of Class III Antiarrhythmic Drug Therapy"; Am. J. Cardiol. 72 (1993), 44 B-49 B). Bei höheren Herzfrequenzen bzw. Stimulation der β-Rezeptoren hingegen ist die das Aktionspotential verlängernde Wirkung der I_{Kr}-Blocker deutlich reduziert, was darauf zurückgeführt wird, daß unter diesen Bedingungen der I_{Ks} stärker zur Repolarisierung beiträgt. Aus diesen Gründen weisen die erfindungsgemäßen Substanzen, die als I_{Ks}-Blocker wirken, wesentliche Vorteile auf gegenüber den bekannten I_{Kr}-Blockern. Inzwischen wurde auch beschrieben, daß eine Korrelation zwischen I_{Ks}-Kanal-inhibitorischer Wirkung und dem Unterbinden von lebensbedrohlichen cardialen Arrhythmien besteht, wie sie beispielsweise durch β-adrenerge Hyperstimulation ausgelöst werden (z. B. T. J. Colatsky, C. H. Follmer und C. F. Starmer; "Channel Specificity in Antiarrhythmic Drug Action; Mechanism of potassium channel block and its role in suppressing and aggravating cardiac arrhythmias"; Circulation 82 (1990), 2235 - 2242; A. E. Busch, K. Malloy, W. J. Groh, M. D. Varnum, J. P. Adelman und J. Maylie; "The novel class III antiarrhythmics NE-10064 and NE-10133 inhibit I_{sK} channels in xenopus oocytes and I_{Ks} in guinea pig cardiac myocytes"; Biochem. Biophys. Res. Commun. 202 (1994), 265 - 270).

Darüber hinaus tragen die Verbindungen zu einer deutlichen Verbesserung der Herzinsuffizienz, insbesondere der Stauungsherzinsuffizienz (Congestive Heart Failure) bei, vorteilhafterweise in der Kombination mit kontraktionsfördernden (positiv inotropen) Wirkstoffen, z.B. Phosphordiesterasehemmern.

Trotz der therapeutisch nutzbaren Vorteile, die durch eine Blockade des I_{Ks} erzielt werden können, sind bisher nur sehr wenige Verbindungen beschrieben, die diesen Subtyp des "delayed rectifiers" hemmen. Die in der Entwicklung befindliche Substanz Azimilid weist zwar auch eine blockierende Wirkung auf den I_{Ks} auf, blockiert jedoch vorwiegend den I_{Kr} (Selektivität 1:10). In der WO-A-95/14470 wird die Verwendung von Benzodiazepinen als selektive Blocker des I_{Ks} beansprucht. Weitere I_{Ks}-Blocker sind beschrieben in FEBS Letters 396 (1996), 271-275: "Specific blockade of slowly activating I_{sK} channels by chromanols..." und Pflügers Arch. - Eur. J. Physiol. 429 (1995), 517-530: "A new class of inhibitors of cAMP-mediated Cl- secretion in rabbit colon, acting by the reduction of cAMP-activated K⁺ conductance". Die Wasserlöslichkeit der dort beschriebenen Verbindungen ist jedoch geringer.

Die erfindungsgemäßen Verbindungen der Formel I und ihre physiologisch verträglichen Salze können somit am Tier, bevorzugt am Säingetier, und insbesondere am Menschen als Arzneimittel für sich allein, in Mischungen untereinander oder in Form von pharmazeutischen Zubereitungen verwendet werden. Gegenstand der vorliegenden Erfindung sind auch die Verbindungen der Formel I und ihre physiologisch verträglichen Salze zur Anwendung als Arzneimittel, ihre Verwendung in der Therapie und Prophylaxe der genannten Krankheitsbilder und ihre Verwendung zur Herstellung von Medikamenten dafür und von Medikamenten mit K⁺-Kanal-blockierender Wirkung. Weiterhin sind Gegenstand der vorliegenden Erfindung pharmazeutische Zubereitungen, die als aktiven Bestandteil eine wirksame Dosis mindestens einer Verbindung der Formel I und/oder eines physiologisch verträglichen Salzes davon neben üblichen, pharmazeutisch einwandfreien Träger- und Hilfsstoffen enthalten. Die pharmazeutischen Zubereitungen enthalten normalerweise 0,1 bis 90 Gewichtsprozent der Verbindungen der Formel I und/oder ihrer physiologisch verträglichen Salze. Die Herstellung der pharmazeutischen Zubereitungen kann in an sich bekannter Weise erfolgen. Dazu werden die Verbindungen der Formel I und/oder ihre physiologisch verträglichen Salze zusammen mit einem oder mehreren festen oder flüssigen galenischen Trägerstoffen und/oder Hilfsstoffen und, wenn gewünscht, in Kombination mit anderen Arzneimittelwirkstoffen in eine geeignete Darreichungsform bzw. Dosierungsform gebracht, die dann als Arzneimittel in der Humanmedizin oder Veterinärmedizin verwendet werden kann.

Arzneimittel, die erfindungsgemäße Verbindungen der Formel I und/oder ihre physiologisch verträglichen Salze enthalten, können oral, parenteral, z. B intravenös, rektal, durch Inhalation oder topisch appliziert werden, wobei die bevorzugte Applikation vom Einzelfall, z. B. dem jeweiligen Erscheinungsbild der zu behandelnden Erkrankung, abhängig ist.

Welche Hilfsstoffe für die gewünschte Arzneimittelformulierung geeignet sind, ist dem Fachmann auf Grund seines Fachwissens geläufig. Neben Lösemitteln, Gelbildnern, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Mittel zur Erzielung eines Depoteffekts, Puffersubstanzen oder Farbstoffe verwendet werden.

Die Verbindungen der Formel I können zur Erzielung einer vorteilhaften therapeutischen Wirkung auch mit anderen Arzneiwirkstoffen kombiniert werden. So sind in der Behandlung von Herz-Kreislauferkrankungen vorteilhafte Kombinationen mit herz-kreislaufaktiven Stoffen möglich. Als derartige, für Herz-Kreislauferkrankungen vorteilhafte Kombinationspartner kommen beispielsweise andere Antiarrhythmika, so Klasse I-, Klasse II- oder Klasse III-Antiarrhythmika, in Frage, wie beispielsweise I_{Kr}-Kanalblocker, z.B. Dofetilid, oder weiterhin blutdrucksenkende Stoffe wie ACE-Inhibitoren (beispielsweise Enalapril, Captopril, Ramipril), Angiotensin-Antagonisten, K⁺-Kanalaktivatoren, sowie alpha- und beta-Rezeptorenblocker, aber auch sympathomimetische und adrenerg wirkende Verbindungen, sowie Na⁺/H⁺-Exchange-Inhibitoren, Calciumkanalantagonisten, Phosphodiesterasehemmer und andere positiv inotrop wirkende Stoffe, wie z. B. Digitalisglykoside, oder Diuretika. Weiterhin sind Kombinationen mit antibiotisch wirkenden Substanzen und mit Antiulkusmitteln vorteilhaft, beispielsweise mit H₂-Antagonisten (z. B. Ranitidin, Cimetidin, Famotidin, etc.), insbesondere bei der Anwendung zur Behandlung von Magen-Darmerkrankungen.

Für eine orale Anwendungsform werden die aktiven Verbindungen mit den dafür geeigneten Zusatzstoffen, wie Trägerstoffen, Stabilisatoren oder inerten Verdünnungsmittel, vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran. Als Lösungsmittel für wäßrige oder alkoholische Lösungen kommen z. B. Wasser, Ethanol oder Zuckerlösungen oder Gemische davon, in Betracht. Weitere Hilfsstoffe, auch für andere Applikationsformen, sind z. B. Polyethylenglykole und Polypropylenglykole.

Zur subkutanen oder intravenösen Applikation werden die aktiven Verbindungen, gewünschtenfalls mit den dafür üblichen Substanzen wie Lösungsvermittlern, Emulgatoren oder weiteren Hilfsstoffen, in Lösung, Suspension oder Emulsion gebracht. Die Verbindungen der Formel I und ihre physiologisch verträglichen Salze können auch lyophilisiert werden und die erhaltenen Lyophilisate z. B. zur Herstellung von Injektions- oder Infusionspräparaten verwendet werden. Als Lösungsmittel kommen z. B. Wasser, physiologische Kochsalzlösung oder Alkohole, z. B. Ethanol, Propanol, Glycerin, in Betracht, daneben auch Zuckerlösungen wie Glucose- oder Mannitlösungen, oder auch Mischungen aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen der Wirkstoffe der Formel I oder ihrer physiologisch verträglichen Salze in einem pharmazeintisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gewichtsprozent.

Die Dosierung des zu verabreichenden Wirkstoffs der Formel I bzw. der physiologisch verträglichen Salze davon hängt vom Einzelfall ab und ist wie üblich für eine optimale Wirkung den Gegebenheiten des Einzelfalls anzupassen. So hängt sie natürlich ab von der Häufigkeit der Verabreichung und von der Wirkstärke und Wirkdauer der jeweils zur Therapie oder Prophylaxe eingesetzten Verbindungen, aber auch von Art und Stärke der zu behandelnden Krankheit sowie von Geschlecht, Alter, Gewicht und individueller Ansprechbarkeit des zu behandelnden Menschen oder Tieres und davon, ob akut oder prophylaktisch therapiert wird. Üblicherweise beträgt die tägliche Dosis einer Verbindung der Formel I bei Verabreichung an einem etwa 75 kg schweren Patienten mindestens 0.001 mg/kg Körpergewicht, vorzugsweise mindestens 0.01 mg/kg Körpergewicht, insbesondere mindestens 0.1 mg/kg Körpergewicht, bis höchstens 100 mg/kg Körpergewicht, vorzugsweise bis höchstens 20 mg/kg Körpergewicht, insbesondere bis höchstens 1 mg/kg Körpergewicht. Die Dosis kann in Form einer Einzeldosis verabreicht werden oder in mehrere, z. B. zwei, drei oder vier Einzeldosen aufgeteilt werden. Insbesondere bei der Behandlung akuter Fälle von Herzrhythmusstörungen, beispielsweise auf einer Intensivstation, kann auch eine parenterale Verabreichung durch Injektion oder Infusion, z. B. durch eine intravenöse Dauerinfusion, vorteilhaft sein.

Die Verbindungen der Formel I und ihre physiologisch verträglichen Salze hemmen selektiv K⁺(cAMP)-Kanäle bzw. I_{Ks}-Kanäle. Aufgrund dieser Eigenschaft können sie außer als Arzneimittelwirkstoffe in der Humanmedizin und Veterinärmedizin auch als wissenschaftliches Tool oder als Hilfsmittel für biochemische Untersuchungen eingesetzt werden, bei denen eine Beeinflussung von Kaliumkanälen beabsichtigt ist, sowie für diagnostische Zwecke, z. B. in der in vitro-Diagnostik von Zell- oder Gewebsproben. Ferner können sie, wie bereits oben erwähnt, als Zwischenprodukte zur Herstellung weiterer Arzneimittelwirkstoffe eingesetzt werden.

Erklärung der im Text verwendeten Abkürzungen
- DMA: Dimethylacetamid
- HMPT: Hexamethylphosphorsäuretriamid
- TMU: Tetramethylharnstoff
- hr: Stunde(n)
- M: Mol
- MCPBA: m-Chlorperbenzoesäure
- mM: Millimol
- min: Minuten
- TEA: Triethylamin
- THF: Tetrahydrofuran
- NBS: N-Brom-Succinimid
- NCS: N-Chlor-Succinimid

### Beispiel 1: Ethansulfonsäure (3-hydroxy-2,2-dimethyl-3,4-dihydro-2H-pyrano[3,2-c]pyridin-4-yl)-methyl-amid

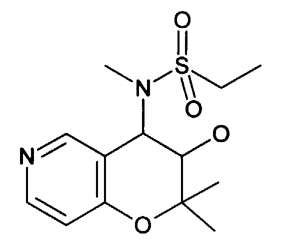

Zu einer Suspension von 27 mg (0,7 mmol) NaH (60%ig) in 1,5 ml DMSO gibt man eine Lösung von 480 mg (3,5 mmol) N-Methyl-ethylsulfonamid in 0,75 ml DMSO (Dimethylsulfoxid). Nach 2 h Rühren bei RT tropft man eine Lösung von 0,48 g (2.7 mmol) 2,2-Dimethyl-1a,7b-dihydro-2H-1,3-dioxa-6-aza-cyclopropa[a]naphthalin (hergestellt analog G.Burell et al. J. Med. Chem. 33 (1990) 3023 - 3027) in 6 ml DMSO zu. Man erhitzt 4 h auf 60°C und läßt anschließend über Nacht bei RT rühren. Die Reaktionsmischung wird auf Eiswasser gegeben und mit Ethylacetat extrahiert. Nach Entfernen des LM im Vakuum wird der erhaltene Feststoff mit einer Mischung aus Heptan und Ethylacetat verrührt und abgesaugt. Man erhält 400 mg (67 %) Ethansulfonsäure (3-hydroxy-2,2-dimethyl-3,4-dihydro-2H-pyrano[3,2-c]pyridin-4-yl)-methyl-amid als Feststoff (Smp. 163°C).

## Patentansprüche

1. Verbindungen der Formel I 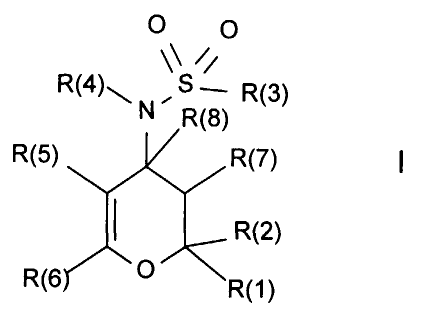 worin bedeuten:
R(1) und R(2)
unabhängig voneinander Wasserstoff, CF₃, C₂F₅, C₃F₇, Alkyl mit 1, 2, 3, 4, 5 oder 6 C-Atomen oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
oder
R(1) und R(2)
gemeinsam eine Alkylenkette mit 2, 3, 4, 5, 6, 7, 8, 9 oder 10 C-Atomen;
wobei eine CH₂-Gruppe der Alkylenkette durch -O-, -CO-, -S-, -SO-, -SO₂- oder -NR(10)- ersetzt sein kann;
R(10) Wasserstoff oder Alkyl mit 1, 2 oder 3 C-Atomen;
R(3) R(12)-CₐH₂ₐ[NR(13)]ₘ-;
R(12) Wasserstoff oder Cycloalkyl mit 3, 4, 5, 6, 7 oder 8 C-Atomen, CF₃, C₂F₅ oder C₃F₇;
a Null, 1, 2, 3, 4, 5, 6, 7, 8, 9 oder 10;
m Null oder 1;
R(13) Wasserstoff oder Alkyl mit 1, 2, 3, 4, 5 oder 6 C-Atomen;
oder
R(12) und R(13)
gemeinsam eine Alkylengruppe mit 4, 5, 6, 7 oder 8 C-Atomen,
wobei eine CH₂-Gruppe der Alkylengruppe durch -O-, -[SO_{Null, 1 oder 2}]-, -CO- oder -NR(10)- ersetzt sein kann; R(10) Wasserstoff oder Alkyl mit 1, 2 oder 3 C-Atomen;
R(4) R(14)-CᵣH₂ᵣ;
r Null, 1, 2, 3, 4, 5, 6, 7, 8, 9, 10, 11, 12, 13, 14, 15, 16, 17, 18, 19 oder 20;
R(14) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
wobei eine CH₂-Gruppe der Gruppe CᵣH₂ᵣ ersetzt sein kann durch -O-, -CH=CH-, -C≡C-, -CO-, -CO-O-, -CO-NR(11)-, -[SO_{Null, 1 oder 2}]- oder -NR(11)-;
R(11) Wasserstoff oder -(CₐH₂ₐ)-R(10);
wobei eine CH₂-Gruppe der Gruppe CₐH₂ₐ ersetzt sein kann durch -O-, -CH=CH-, -C≡C-, -CO-, -CO-O-, -O-CO-, -S-, -SO-, -SO₂-, NR(10)- oder -CONR(10)-;
R(10) Wasserstoff oder Alkyl mit 1, 2 oder 3 C-Atomen;
oder
R(3) und R(4)
gemeinsam eine Alkylenkette mit 3, 4, 5, 6, 7 oder 8 C-Atomen,
wobei eine CH₂-Gruppe der Alkylenkette durch -O-, -[SO_{Null, 1 oder 2}]-, -CO- oder -NR(11)- ersetzt sein kann;
R(11) Wasserstoff oder -(CₐH₂ₐ)-R(10),
wobei eine CH₂-Gruppe der Gruppe CₐH₂ₐ ersetzt sein kann durch -O-, -CH=CH-, -C≡C-, -CO-, -CO-O-, -O-CO-, -S-, -SO-, -SO₂-, NR(10)- oder -CONR(10)-;
R(10) Wasserstoff oder Alkyl mit 1, 2 oder 3 C-Atomen;
R(5) und R(6)
-CR(15)=CR(16)-CR(17)=N-,
-CR(15)=CR(16)-N=CR(17)-,
-CR(15)=N-CR(17)=N-,
-CR(15)=N-N=CR(17)-,
-N=CR(16)-CR(17)=N- oder
-S-CR(15)=CR(16)-;
R(15), R(16) und R(17)
unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, CN, CF₃, C₂F₅, C₃F₇, N₃, NO₂, -CONR(19)R(21), -COOR(21),
R(22)-CₛH₂ₛ-Z- oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
R(19) Wasserstoff oder Alkyl mit 1, 2 oder 3 C-Atomen;
R(21) Wasserstoff, Methyl, Ethyl, Phenyl oder
-CᵤH₂ᵤ-NR(19)R(20);
wobei das Phenyl unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
R(20)
Wasserstoff oder Alkyl mit 1, 2 oder 3 C-Atomen;
u 2 oder 3;
R(22) Wasserstoff, Cycloalkyl mit 3, 4, 5, 6, 7 oder 8 C-Atomen,
-COOR(21), CONR(19)R(21), Thienyl, Imidazolyl, Pyridyl, Chinolyl, Isochinolyl, Piperidyl, 1-Pyrrolidinyl, N-Morpholino, N-Methylpiperazino, CF₃, C₂F₅, C₃F₇ oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
s Null, 1, 2, 3, 4, 5 oder 6;
Z -[S(O)_{Null, 1 oder 2}]-, -CO-, -SO_{(0, 1 oder 2)}-NR(11)-, -SO₂-O-, -O-, -NR(11)- oder -[CO-NR(11)]-;
R(7) Wasserstoff, Hydroxy, Alkoxy mit 1, 2, 3 oder 4 C-Atomen, Acyloxy mit 1, 2, 3 oder 4 C-Atomen, Cl, Br, F, Alkyl mit 1, 2, 3 oder 4 C-Atomen;
R(8) Wasserstoff oder Alkyl mit 1, 2, 3, 4, 5 oder 6 C-Atomen;
und deren physiologisch verträgliche Salze.

2. Verbindungen der Formel I nach Anspruch 1, in welcher bedeuten:
R(1) und R(2)
unabhängig voneinander Wasserstoff, CF₃, C₂F₅, C₃F₇, Alkyl mit 1, 2, 3, 4, 5 oder 6 C-Atomen oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
oder
R(1) und R(2)
gemeinsam eine Alkylenkette mit 2, 3, 4, 5, 6, 7, 8, 9 oder 10 C-Atomen;
wobei eine CH₂-Gruppe der Alkylenkette durch -O-, -CO-, -S-, -SO-, -SO₂- oder -NR(10)- ersetzt sein kann;
R(10) Wasserstoff oder Alkyl mit 1, 2 oder 3 C-Atomen;
R(3) R(12)-CₐH₂ₐ[NR(13)]ₘ-;
R(12) Wasserstoff oder Cycloalkyl mit 3, 4, 5, 6, 7 oder 8 C-Atomen,
CF₃, C₂F₅ oder C₃F₇;
a Null, 1, 2, 3, 4, 5, 6, 7, 8, 9 oder 10;
m Null oder 1;
R(13) Wasserstoff oder Alkyl mit 1, 2, 3, 4, 5 oder 6 C-Atomen;
R(4) R(14)-CᵣH₂ᵣ;
r Null, 1, 2, 3, 4, 5, 6, 7, 8, 9, 10, 11, 12, 13, 14, 15, 16, 17, 18, 19 oder 20;
R(14) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
wobei eine CH₂-Gruppe der Gruppe CᵣH₂ᵣ ersetzt sein kann durch -O-, -CH=CH-, -C≡C-, -CO-, -CO-O-, -CO-NR(11)-, -[SO_{Null, 1 oder 2}]- oder -NR(11)-;
R(11) Wasserstoff oder -(CₐH₂ₐ)-R(10)
wobei eine CH₂-Gruppe der Gruppe CₐH₂ₐ ersetzt sein kann durch -O-, -CH=CH-, -C≡C-, -CO-, -CO-O-, -O-CO-, -S-,-SO-, -SO₂-, NR(10)- oder -CONR(10)-;
a Null, 1, 2, 3, 4, 5, 6, 7, 8, 9 oder 10;
R(5) und R(6)
-CR(15)=CR(16)-CR(17)=N-,
-CR(15)=CR(16)-N=CR(17)-,
-CR(15)=N-CR(17)=N-,
-CR(15)=N-N=CR(17)-,
-N=CR(16)-CR(17)=N- oder
-S-CR(15)=CR(16)-;
R(15), R(16), R(17)
unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, CN, CF₃, C₂F₅, C₃F₇, N₃, NO₂, -CONR(19)R(21), -COOR(21), R(22)-CₛH₂ₛ-Z- oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
R(19) Wasserstoff oder Alkyl mit 1, 2 oder 3 C-Atomen;
R(21) Wasserstoff, Methyl, Ethyl, Phenyl oder
-CᵤH₂ᵤ-NR(19)R(20);
u 2 oder 3;
R(20) Wasserstoff oder Alkyl mit 1, 2 oder 3 C-Atomen;
wobei das Phenyl unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
R(22) Wasserstoff, Cycloalkyl mit 3, 4, 5, 6, 7 oder 8 C-Atomen,
-COOR(21), CONR(19)R(21), Thienyl, Imidazolyl, Pyridyl, Chinolyl, Isochinolyl, Piperidyl, 1-Pyrrolidinyl, N-Morpholino, N-Methylpiperazino, CF₃, C₂F₅ oder C₃F₇ oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
s Null, 1, 2, 3, 4, 5 oder 6;
Z -[S(O)_{Null, 1 oder 2}], -CO-, -SO₂-NR(11)-, -SO₂-O-, -O-, -NR(11)- oder-[CO-NR(11)]-;
R(7) Wasserstoff, Hydroxy, Alkoxy mit 1, 2, 3 oder 4 C-Atomen, Acyloxy mit 1, 2, 3 oder 4 C-Atomen, Cl, Br, F, Alkyl mit 1, 2, 3 oder 4 C-Atomen;
R(8) Wasserstoff oder Alkyl mit 1, 2, 3, 4, 5 oder 6 C-Atomen;
und deren physiologisch verträgliche Salze

3. Verbindung der Formel I nach Anspruch 1 oder 2, in der bedeuten:
R(1) und R(2)
unabhängig voneinander Wasserstoff, CF₃, C₂F₅, C₃F₇, Alkyl mit 1, 2, 3, 4, 5 oder 6 C-Atomen oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
oder
R(1) und R(2)
gemeinsam eine Alkylenkette mit 2, 3, 4, 5, 6, 7, 8, 9 oder 10 C-Atomen;
wobei eine CH₂-Gruppe der Alkylenkette durch -O-, -CO-, -S-, -SO-, -SO₂- oder -NR(10)- ersetzt sein kann;
R(10) Wasserstoff oder Alkyl mit 1, 2 oder 3 C-Atomen;
R(3) R(12)-CₐH₂ₐ[NR(13)]ₘ-;
R(12) Wasserstoff oder Cycloalkyl mit 3, 4, 5, 6, 7 oder 8 C-Atomen,
CF₃, C₂F₅ oder C₃F₇;
a Null, 1, 2, 3, 4, 5, 6, 7, 8, 9 oder 10;
m Null oder 1;
R(13) Wasserstoff oder Alkyl mit 1, 2, 3, 4, 5 oder 6 C-Atomen;
R(4) R(14)-CᵣH₂ᵣ;
r 1, 2, 3, 4, 5, 6, 7, 8, 9, 10, 11, 12, 13, 14, 15, 16, 17, 18, 19 oder 20;
R(14) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
wobei eine CH₂-Gruppe der Gruppe CᵣH₂ᵣ ersetzt sein kann durch -O-, -CH=CH-, -C≡C-, -CO-, -CO-O-, -CO-NR(11)-, -[SO_{Null, 1 oder 2}]- oder -NR(11)-;
R(11) Wasserstoff oder -(CₐH₂ₐ)-R(10),
wobei eine CH₂-Gruppe der Gruppe CₐH₂ₐ ersetzt sein kann durch -O-, -CH=CH-, -C≡C-, -CO-, -CO-O-, -O-CO-, -S-, -SO-, -SO₂-, NR(10)- oder -CONR(10)-;
R(5) und R(6)
-CR(15)=CR(16)-CR(17)=N- oder
-CR(15)=CR(16)-N=CR(17)-;
R(15), R(16), R(17)
unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, CN, CF₃, C₂F₅, C₃F₇, N₃, NO₂, -CONR(19)R(21), -COOR(21),
R(22)-CₛH₂ₛ-Z- oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
R(19) Wasserstoff oder Alkyl mit 1, 2 oder 3 C-Atomen;
R(21) Wasserstoff, Methyl, Ethyl, Phenyl oder
-CᵤH₂ᵤ-NR(19)R(20);
u 2 oder 3;
R(20) Wasserstoff oder Alkyl mit 1, 2 oder 3 C-Atomen;
wobei das Phenyl unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
R(22) Wasserstoff, Cycloalkyl mit 3, 4, 5, 6, 7 oder 8 C-Atomen,
- COOR(21), CONR(19)R(21), Thienyl, Imidazolyl, Pyridyl, Chinolyl, Isochinolyl, Piperidyl, 1-Pyrrolidinyl, N-Morpholino, N-Methylpiperazino, CF₃, C₂F₅ oder C₃F₇ oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
s Null, 1, 2, 3, 4, 5 oder 6;
Z - [S(O)_{Null, 1 oder 2}]-, -CO-, -SO2-NR(11)-, -SO2-O-, -O-, -NR(11)- oder -[CO-NR(11)]-;
R(7) Wasserstoff, Hydroxy, Alkoxy mit 1, 2, 3 oder 4 C-Atomen, Alkyl mit 1, 2, 3 oder 4 C-Atomen;
R(8) Wasserstoff oder Alkyl mit 1, 2, 3, 4, 5 oder 6 C-Atomen;
und deren physiologisch verträgliche Salze

4. Verbindungen der Formel I nach Ansprüchen 1, 2 oder 3, in der bedeuten:
R(1) und R(2)
unabhängig voneinander Wasserstoff, CF₃, C₂F₅, C₃F₇, Alkyl mit 1, 2, 3, 4, 5 oder 6 C-Atomen oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
oder
R(1) und R(2)
gemeinsam eine Alkylenkette mit 2, 3, 4, 5, oder 6 C-Atomen;
wobei eine CH₂-Gruppe der Alkylenkette durch -O-, -CO-, -S-, -SO-, -SO₂- oder -NR(10)- ersetzt sein kann;
R(10) Wasserstoff oder Alkyl mit 1, 2 oder 3 C-Atomen;
R(3) R(12)-CₐH₂ₐ-;
R(12) Wasserstoff oder Cycloalkyl mit 3, 4, 5, 6, 7 oder 8 C-Atomen,
CF₃, C₂F₅ oder C₃F₇;
a Null, 1, 2, 3, 4, 5, 6, 7, 8, 9 oder 10;
R(4) R(14)-CᵣH₂ᵣ;
r 1, 2, 3, 4, 5, 6, 7, 8, 9, 10, 11, 12, oder 13;
R(14) Wasserstoff, Cycloalkyl mit 3, 4, 5, 6, 7 oder 8 C-Atomen, Piperidyl, 1-Pyrrolidinyl, N-Morpholino, N-Methylpiperazino, CF₃, C₂F₅, C₃F₇, Pyridyl, Thienyl, Imidazolyl oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl, Methylsulfonyl und Methylsulfonylamino;
wobei eine CH₂-Gruppe der Gruppe CᵣH₂ᵣ ersetzt sein kann durch -O-, -CH=CH-, -C≡C-, -CO-, -CO-O-, -CO-NR(11)-, -[SO_{Null, 1 oder 2}]- oder -NR(11)-;
R(11) Wasserstoff oder -(CₐH₂ₐ)-R(10)
wobei eine CH₂-Gruppe der Gruppe CₐH₂ₐ ersetzt sein kann durch -O-, -CH=CH-, -C≡C-, -CO-, -CO-O-, -O-CO-, -S-, -SO-, -SO₂-, NR(10)- oder -CONR(10)-;
R(10) Wasserstoff oder Alkyl mit 1, 2 oder 3 C-Atomen;
R(5) und R(6)
-CR(15)=CR(16)-CR(17)=N- oder
-CR(15)=CR(16)-N=CR(17)-;
R(15), R(16), R(17)
unabhängig voneinander Wasserstoff, F, Cl, Br, I, Alkyl mit 1, 2, 3 oder 4 C-Atomen, Cycloalkyl mit 3, 4, 5, 6, 7 oder 8 C-Atomen, CN, CF₃, C₂F₅, C₃F₇, N₃, NO₂, -CONR(19)R(21), -COOR(21),
R(22)-CₛH₂ₛ-Z- oder Phenyl,
das unsubstituiert ist oder substituiert mit 1 oder 2 Substituenten ausgewählt aus der Gruppe bestehend aus F, Cl, Br, I, CF₃, Methyl, Methoxy, Sulfamoyl und Methylsulfonyl;
R(19)
Wasserstoff oder Alkyl mit 1, 2 oder 3 C-Atomen;
R(21) Wasserstoff, Methyl, Ethyl oder Phenyl,
wobei das Phenyl unsubstituiert ist oder substituiert mit 1 oder 2 Substituenten ausgewählt aus der Gruppe bestehend aus F, Cl, Br, I, CF₃, Methyl, Methoxy, Sulfamoyl oder Methylsulfonyl;
R(22) Wasserstoff;
s Null, 1, 2, 3, 4, 5 oder 6;
Z -[S(O)_{Null, 1 oder 2}]-, -CO-, -SO2-NR(11)-, -SO2-O-, -O-, -NR(11)- oder -[CO-NR(11)]-;
R(7) Wasserstoff, Hydroxy, Alkoxy mit 1, 2, 3 oder 4 C-Atomen, Alkyl mit 1, 2, 3 oder 4 C-Atomen;
R(8) Wasserstoff oder Alkyl mit 1, 2, 3, 4, 5 oder 6 C-Atomen;
und deren physiologisch verträgliche Salze.

5. Verfahren zur Herstellung von Verbindungen der Formel I gemäß einem oder mehreren der Ansprüche 1 bis 4, dadurch gekennzeichnet, daß man
a) eine Verbindung der Formel II 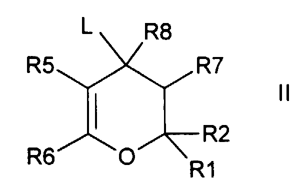 worin R(1), R(2), R(5), R(6), R(7) und R(8) die angegebene Bedeutung besitzen und L eine übliche nucleofuge Fluchtgruppe, insbesondere F, Cl, Br, I, MeSO2-O-, einen p-Toluolsulfonyloxy-Rest, oder R(7) und L gemeinsam ein Epoxidring sind, bedeutet,
mit einem Sulfonamid oder dessen Salz der Formel III 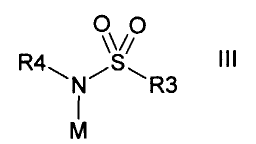 in an sich bekannter Weise umsetzt, worin R(3) und R(4) die angegebene Bedeutung besitzen und M für Wasserstoff oder vorzugsweise für ein Metallatom, besonders bevorzugt für Lithium, Natrium oder Kalium steht;
oder daß man
b) eine Verbindung der Formel IV 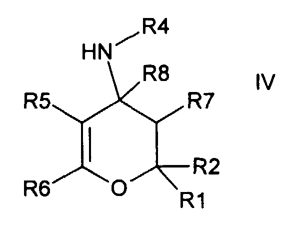 worin R(1), R(2), R(4), R(5), R(6), R(7) und R(8) die angegebene Bedeutung besitzen,
mit einem Sulfonsäure-Derivat der Formel V 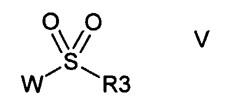 umsetzt, worin R(3) die angegebene Bedeutung besitzt und W eine nucleofuge Fluchtgruppe, wie Fluor, Brom, 1-Imidazolyl, insbesondere aber Chlor bedeutet;
oder daß man
c) eine Verbindung der Formel VI 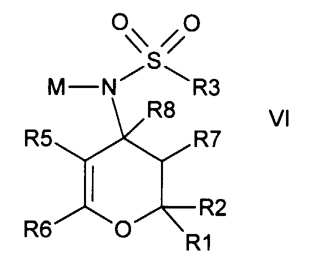 worin R(1), R(2), R(3), R(5), R(6), R(7), R(8), und M die angegebene Bedeutung besitzen, in an sich bekannter Weise mit einem Alkylierungsmittel der Formel VII
R(4)-L VII
im Sinne einer Alkylierungsreaktion umsetzt, worin R(4) mit Ausnahme von Wasserstoff sowie L die angegebene Bedeutung besitzen;
oder daß man
d) in einer Verbindung der Formel I 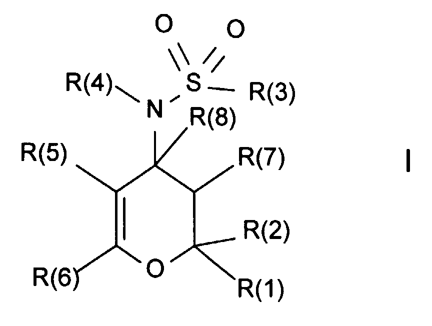 worin R(1) bis R(4), R(7) und R(8) die angegebene Bedeutung besitzen, in mindestens einer der Positionen R(15), R(16), R(17) des Ringsystems R(5)-R(6) eine elektrophile Substitutionsreaktion durchführt, sofern diese Position Wasserstoff bedeutet und die restlichen Substituenten R(1) bis R(8) die angegebene Bedeutung besitzen.

6. Verbindungen der Formel I gemäß einem oder mehreren der Ansprüche 1 bis 4 und ihre physiologisch verträglichen Salze zur Anwendung als Arzneimittel.

7. Pharmazeutische Zubereitung, enthaltend eine wirksame Menge mindestens einer Verbindung der Formel I nach einem oder mehreren der Ansprüche 1 bis 4 und/oder eines physiologisch verträglichen Salzes davon als Wirkstoff, zusammen mit pharmazeutisch annehmbaren Träger- und Zusatzstoffen und gegebenenfalls noch einem oder mehreren anderen pharmakologischen Wirkstoffen.

8. Verwendung einer Verbindung der Formel I nach einem oder mehreren der Ansprüche 1 bis 4 und/oder eines physiologisch verträglichen Salzes davon zur Herstellung eines Medikaments mit K⁺-Kanal-blockierender Wirkung zur Therapie und Prophylaxe von K⁺-Kanal mediierten Krankheiten.

9. Verwendung einer Verbindung der Formel I nach einem oder mehreren der Ansprüche 1 bis 4 und/oder eines physiologisch verträglichen Salzes davon zur Herstellung eines Medikaments zur Therapie oder Prophylaxe von Herzrhythmusstörungen, die durch Aktionspotential-Verlängerung behoben werden können.

10. Verwendung einer Verbindung I nach Anspruch 1 zur Herstellung eines Medikaments zur Behandlung oder Prophylaxe von atrieller Fibrillation oder atriellem Flattern.

11. Verwendung einer Verbindung der Formel I nach einem oder mehreren der Ansprüche 1 bis 4 und/oder eines physiologisch verträglichen Salzes davon zur Herstellung eines Medikaments zum Inhibieren der Magensäuresekretion.

12. Verwendung einer Verbindung der Formel I nach einem oder mehreren der Ansprüche 1 bis 4 und/oder eines physiologisch verträglichen Salzes davon zur Herstellung eines Medikaments zur Therapie oder Prophylaxe von Ulcera des Magens oder des intestinalen Bereiches.

13. Verwendung einer Verbindung der Formel I nach einem oder mehreren der Ansprüche 1 bis 4 und/oder eines physiologisch verträglichen Salzes davon zur Herstellung eines Medikaments zur Therapie oder Prophylaxe der Refluxösophagitis.

14. Verwendung einer Verbindung der Formel I nach einem oder mehreren der Ansprüche 1 bis 4 und/oder eines physiologisch verträglichen Salzes davon zur Herstellung eines Medikaments zur Therapie oder Prophylaxe von Durchfallerkrankungen.

15. Verwendung einer Verbindung der Formel I nach einem oder mehreren der Ansprüche 1 bis 4 und/oder eines physiologisch verträglichen Salzes davon zur Herstellung eines Medikaments zur Therapie oder Prophylaxe aller Typen von Arrhythmien, einschließlich atrialer, ventrikulärer und supraventrikulärer Arrhythmien.

16. Verwendung einer Verbindung der Formel I nach einem oder mehreren der Ansprüche 1 bis 4 und/oder eines physiologisch verträglichen Salzes davon zur Herstellung eines Medikaments zur Therapie oder Prophylaxe von Reentry-Arrhythmien oder zur Verhinderung des plötzlichen Herztodes infolge von Kammerflimmern.

17. Verwendung einer Verbindung der Formel I nach einem oder mehreren der Ansprüche 1 bis 4 und/oder eines physiologisch verträglichen Salzes davon zur Herstellung eines Medikaments zur Therapie der Herzinsuffizienz, insbesondere der Stauungsherzinsuffizienz (Congestive Heart Failure).

18. Verwendung einer Verbindung der Formel I nach einem oder mehreren der Ansprüche 1 bis 4 und/oder eines physiologisch verträglichen Salzes davon zum Blockieren des Kaliumkanals, der durch cyclisches Adenosinmonophosphat (cAMP) geöffnet wird.

19. Verwendung einer Verbindung der Formel I nach einem oder mehreren der Ansprüche 1 bis 4 und/oder eines physiologisch verträglichen Salzes davon zum Herstellen eines Medikaments zum Inhibieren der stimulierten Magensäuresekretion, zur Therapie oder Prophylaxe von Ulcera des Magens oder des intestinalen Bereiches, der Refluxösophagitis, von Durchfallerkrankungen, zur Therapie oder Prophylaxe von Arrhythmien, einschließlich atrialer, ventrikulärer und supraventrikulärer Arrhythmien` atrialer Fibrillation und atrialem Flattern und von Reentry-Arrhythmien, oder zur Verhinderung des plötzlichen Herztodes infolge von Kammerflimmern.

20. Heilmittel, enthaltend eine wirksame Menge einer Verbindung der Formel I nach Ansprüchen 1 bis 4.
